# EUROPEAN PATENT SPECIFICATION

(11) **EP 3 937 790 B1**
(45) Date of publication and mention of the grant of the patent: **09.10.2024**
(21) Application number: 20714348.8
(22) Date of filing: 18.02.2020
(51) Int. Cl.: A61B 7/04, A61B 8/00, A61B 8/08, A61F 2/24, A61N 1/362, A61N 1/39, A61B 8/04

(54) **PROSTHETIC HEART VALVE ASSESSMENT USING HEART SOUNDS**
HERZKLAPPENBEURTEILUNG UNTER VERWENDUNG VON HERZGERÄUSCHEN
ÉVALUATION DE VALVULE CARDIAQUE PROTHÉTIQUE À L'AIDE DE SONS CARDIAQUES

(30) Priority: 12.03.2019 US 201962817324 P
(43) Date of publication of application: 19.01.2022
(73) Proprietor: Cardiac Pacemakers, Inc., St. Paul, Minnesota 55112-5798 (US)
(72) Inventor: AN, Qi, Blaine, Minnesota 55449 (US); THAKUR, Pramodsingh Hirasingh, Woodbury, Minnesota 55129 (US); SHUTE, Jonathan Bennett, Minnetonka, Minnesota 55305 (US); MAILE, Keith R., New Brighton, MN 55112 (US)
(74) Representative: Pfenning, Meinig & Partner mbB
(86) International application number: PCT/US2020/018625
(87) International publication number: WO 2020/185359

(56) References cited:
- US-A1- 2012 296 228
- US-A1- 2013 053 913
- US-A1- 2014 277 239
- US-A1- 2015 126 883
- US-A1- 2017 258 585
- US-B2- 7 736 319

## Description

### CLAIM OF PRIORITY

This application claims the benefit of priority under 35 U.S.C. § 119(e) of U.S. Provisional Patent Application Serial Number 62/817,324, filed on March 12, 2019.

### TECHNICAL FIELD

This document relates generally to medical systems, and more particularly, to systems, devices and methods for monitoring and evaluating function of an implanted prosthetic heart valve in a patient.

### BACKGROUND

More than a million people in the United States have aortic stenosis, a degenerative valvular disease that worsens over time. The aortic valvular orifice is normally capable of opening during systole in a range of 4 to 6 square centimeters, therefore allowing free ejection of the ventricular blood volume into the aorta. In patients with aortic stenosis, aortic valve does not open fully because of thickening of the valve leaflets. The aortic valvular orifice can become tightly stenosed, such that the blood cannot be adequately ejected from the left ventricle to the aorta. As a result of a markedly increase in the left ventricular pressure to force open the stenosed aortic orifice, the heart compensates by thickening its walls to maintain sufficient pumping pressure. Without proper treatment, cardiac function may substantially deteriorate. An early symptom of aortic stenosis is shortness of breath associated with activity. Advanced symptoms can include syncope, chest pain and heart failure.

While some patients with aortic stenosis are treated with medication, patients with severe aortic stenosis often requires more extensive treatment, such as surgical placement of a prosthetic heart value (PHV). A PHV can include a mechanical device or a bioprosthetic valve (such as obtained from porcine or bovine pericardium fashioned leaflets) that replaces a diseased natural heart valve, and fulfils the function of gating the blood flow between heart chambers, or between a heart chamber and an associated major blood vessel. Examples of the PHV may include prosthetic aortic valve, prosthetic mitral valve, prosthetic tricuspid valve, and prosthetic pulmonary valve. A PHV may be implanted, for example, via an open-heart surgery, or a transcatheter approach. In the realm of aortic value replacement, these are known as surgical aortic valve replacement (SAVR), or a transcatheter aortic valve replacement (TAVR). During a SAVR procedure, an incision may be made in the chest to access the heart. The diseased aortic valve may then be removed and replaced with a prosthetic aortic valve. TAVR is a minimally invasive, catheter-based procedure for severe aortic stenosis patients. During a TAVR procedure, a catheter may be inserted into and pass through an artery in the groin or a small incision between the ribs. A prosthetic aortic valve may be compressed and fed through the catheter until it reaches a patient natural aortic valve. The prosthetic value can then be expanded, by a balloon or a self-expanding nitinol cage, within the patient's stenotic aortic valve. The catheter may then be removed. The prosthetic valve replaces the diseased natural valve, increasing blood flow throughout the body.

Some patients may experience adverse events following valve replacement surgery. SAVR and TAVR have been reported to have different patterns of adverse events. For example, SAVR may be associated with acute kidney injury, atrial fibrillation, and transfusion requirements. TAVR may have a relatively higher rate of residual aortic regurgitation and a need for pacemaker implantation in some patients. Bioprosthetic valve failure, which involves aortic valve reintervention, severe hemodynamic structural valve deterioration, heart failure, or valve-related death, can occur in some patients. Frequent evaluation of PHV function and viability can help timely detect valve dysfunction or failure, thereby preventing or reducing adverse events associated with valve replacement.

Document US 2017/258585 A1 discloses sensor-integrated prosthetic valves that include a plurality of valve leaflets, a frame assembly configured to support the plurality of valve leaflets and define a plurality of commissure supports terminating at an outflow end of the prosthetic valve, a sensor device associated with the frame assembly and configured to generate a sensor signal, for example, a sensor signal indicating deflection of one or more of the plurality of commissure supports, and a transmitter assembly configured to receive the sensor signal from the sensor device and wirelessly transmit a transmission signal that is based at least in part on the sensor signal.

Document US 2013/053913 A1 discloses a method to determine pacing parameters for an implantable medical device (IMD) and to collect heart sounds during the cardiac cycles. The method comprises changing a value for a pacing parameter between the cardiac cycles and analyzing a characteristic of interest from the heart sounds. The method comprises setting a desired value for the pacing parameter based on the characteristic of interest from the heart sounds. A system comprises inputs configured to be coupled to at least one lead having electrodes to sense intrinsic events and to deliver pacing pulses over cardiac cycles. The system has a sensor for collecting heart sounds during cardiac cycles and controller to control delivery of pacing pulses based on pacing parameters. The controller changes a value for at least one of the pacing parameters between the cardiac cycles and provides an analysis module to analyze a characteristic of interest from the heart sounds.

US 2012/296228 A1 describes an implantable medical device that receives both heart sound and electrogram signals. A processor within the implantable medical device extracts physiologically relevant information from both the heart sound signal and the electrogram signal. Based on the extracted physiologically relevant information a set of pacing parameters is evaluated. The values of the pacing parameters may be changed by the implantable medical device in response to the physiologically relevant information extracted from the heart sound signal and the electrogram signal.

### OVERVIEW

The present document discusses systems, devices, and methods for evaluating the function and viability of an implanted PHV using information of a heart, such as cardiac acceleration information sensed from the patient. According to an embodiment discussed herein, a medical-device system can comprise a sensor circuit and a PHV monitor circuit. The sensor circuit can receive acceleration information from the patient. The acceleration information may be indicative of functioning of a PHV implanted in a patient, such as a prosthetic aortic valve. The acceleration information may include vibrational or acoustic information, such as heart sounds (HS) information. The PHV monitor circuit can generate a HS metric using the received acceleration information, and produce an indicator of PHV function using the HS metric. An output circuit can generate an alert if the PHV function indicator indicates PHV dysfunction. The PHV function may be monitored during a valve replacement procedure (e.g., SAVR or TAVR). In an example, peri-operative monitoring of HS produced by PHV can assist in identifying an optimal PHV implant site where a desired hemodynamic effect can be achieved. Additionally or alternatively, the PHV function may be evaluated during post-operative recovery and thereafter. According to some embodiments, the present system and method may be used to determine patient candidacy for valve replacement surgeries, such as by using patient natural HS information produced by a natural heart valve. A risk indicator may be provided to a clinician indicating patient natural valve function and a need for heart valve repair or replacement.

Example 1 is a medical-device system that comprises a sensor circuit and a prosthetic heart valve (PHV) monitor circuit. The sensor circuit can be configured to receive acceleration information from a patient. The acceleration information may be indicative of functioning of a PHV implanted in a patient. The acceleration information may include vibrational or acoustic information. The PHV monitor circuit can be configured to generate a heart sound (HS) metric using the received acceleration information, and to generate an indicator of PHV function using the generated HS metric. In an example, the acceleration information can include HS information of a heart of the patient.

In Example 2, the subject matter of Example 1 optionally includes the acceleration information that may include HS information from the patient.

In Example 3, the subject matter of any one or more of Examples 1-2 optionally includes the PHV that can include a bioprosthetic aortic valve implanted surgically in the patient or via a transcatheter approach.

In Example 4, the subject matter of any one or more of Examples 1-3 optionally includes the HS metric that an include a second heart sound (S2) intensity.

In Example 5, the subject matter of any one or more of Examples 1-4 optionally includes the HS metric that can include a first heart sound (S1) intensity.

In Example 6, the subject matter of any one or more of Examples 1-5 optionally includes the HS metric that can include a HS ratio of a first heart sound (S1) intensity to a second heart sound (S2) intensity.

In Example 7, the subject matter of any one or more of Examples 1-6 optionally includes the PHV monitor circuit that may be configured to generate the indicator indicating PHV dysfunction in responds to the generated HS metric exceeding a threshold or being outside of a specific range.

In Example 8, the subject matter of any one or more of Examples 1-7 optionally includes the received HS information that can include a HS signal sensed from the patient. The PHV monitor circuit can be configured to filter the HS signal using a filter circuit with a specified frequency passband; generate the HS metric including signal energy of the filtered HS signal, and generate the indicator indicating PHV dysfunction in responds to the generated signal energy of the filtered HS signal exceeding a threshold.

In Example 9, the subject matter of Example 8 optionally includes the filer circuit having a frequency passband with a lower cutoff of substantially 100 Hz.

In Example 10, the subject matter of any one or more of Examples 1-9 optionally includes an output circuit configured to generate an alert if the PHV function indicator indicates a PHV dysfunction.

In Example 11, the subject matter of any one or more of Examples 1-10 optionally includes the output circuit that can be configured to generate an indication of position adjustment of the PHV during a heart valve replacement procedure using the generated HS metric.

In Example 12, the subject matter of any one or more of Examples 1-11 optionally includes the PHV monitor circuit that can be configured to generate a trend of the HS metric over time post implant of the PHV, where the system comprises an output circuit configured to generate an alert of delayed post-implant recovery using the generated HS metric trend.

In Example 13, the subject matter of any one or more of Examples 1-12 optionally includes a heart failure detector circuit that can be configured to detect worsening heart failure (WHF) in a patient using the received HS information.

In Example 14, the subject matter of Example 13 optionally includes the heart failure detector circuit that can be configured to detect WHF attributable to PHV dysfunction in response to an increase in S3 intensity and a decrease in S2 intensity from their respective baselines.

In Example 15, the subject matter of any one or more of Examples 1-14 optionally includes the sensor circuit coupled to an accelerometer sensor mounted on a catheter used for delivering the PHV to a target implant site and configured to sense the HS information from the patient.

In Example 16, the subject matter of any one or more of Examples 1-15 optionally includes an ambulatory medical device (AMD) including the accelerometer sensor, the sensor circuit, and the PHV monitor circuit.

In Example 17, the subject matter of any one or more of Examples 1-16 optionally includes a risk stratifier circuit, and the HS information includes natural HS information generated by a natural heart valve of the patient. The risk stratifier circuit can be configured to generate a natural HS metric using the natural HS information, and generate a risk indicator using the generated natural HS metric, the risk indicator indicating patient candidacy for heart valve replacement.

Example 18 is a method of assessing a prosthetic heart valve (PHV) implanted in a patient. The method comprises steps of: receiving heart sound (HS) information from a patient, the HS information indicative of functioning of the PHV; generating a HS metric using the received HS information; and generating an indicator of PHV function using the generated HS metric.

In Example 19, the subject matter of Example 18 optionally includes the HS metric that can include one or more of: a first heart sound (S1) intensity; a second heart sound (S2) intensity; or a HS ratio of an S 1 intensity to an S2 intensity.

In Example 20, the subject matter of any one or more of Example 18-19 optionally includes generating the indicator including generating a PHV dysfunction indicator in responds to the generated HS metric exceeding a threshold or being outside of a specific range.

In Example 21, the subject matter of any one or more of Examples 18-20 optionally includes steps of: filtering the HS signal using a filter circuit with a specified frequency passband; generating the HS metric including signal energy of the filtered HS signal; and generating the indicator indicating PHV dysfunction in responds to the generated signal energy of the filtered HS signal exceeding a threshold.

In Example 22, the subject matter of any one or more of Examples 18-21 optionally includes generating an indication of position adjustment of the PHV during a heart valve replacement procedure using the generated HS metric.

In Example 23, the subject matter of any one or more of Examples 18-22 optionally includes steps of generating a trend of the HS metric over time post implant of the PHV, and generating an alert of delayed post-implant recovery using the generated HS metric trend.

In Example 24, the subject matter of any one or more of Examples 18-23 optionally include detecting detect WHF attributable to PHV dysfunction in response to an increase in S3 intensity and a decrease in S2 intensity from their respective baselines.

In Example 25, the subject matter of any one or more of Examples 18-24 optionally includes steps of: receiving natural HS information generated by a natural heart valve of the patient; generating a natural HS metric using the natural HS information; and generating a risk indicator using the generated natural HS metric, the risk indicator indicating patient candidacy for heart valve replacement.

This Overview is an overview of some of the teachings of the present application and not intended to be an exclusive or exhaustive treatment of the present subject matter. Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present disclosure is defined by the appended claims and their legal equivalents.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1 illustrates generally an example of a patient management system and portions of an environment in which the system may operate.
FIG. 2 illustrates generally an example of a PHV monitoring system configured to monitor and assess function of an implanted PHV.
FIG. 3 illustrates by way of example portions of a system that generate a PHV indicator using various heart sound metrics.
FIGS. 4A-4B are graphs illustrating changes in heart sounds of a patient subsequent to a TVAR procedure.
FIG. 5 illustrates generally an example of a patient monitoring system configured to monitor and assess patient natural valve function.
FIG. 6 is a flow chart illustrating an example of a method for assessing a PHV implanted in a patient.
FIG. 7 illustrates generally a block diagram of an example machine upon which any one or more of the techniques (e.g., methodologies) discussed herein may perform.

### DETAILED DESCRIPTION

This document describes system and methods for monitoring and evaluating the function of a prosthetic heart valve (PHV) implanted in a patient, such as a bioprosthetic aortic valve implanted via a SAVR or TAVR procedure. In an example, an ambulatory medical device (AMD) can monitor acceleration information of a heart of the patient, such as heart sounds (HS) produced by the PHV. Examples of the AMD can include an implantable medical device (IMD), a subcutaneous medical device, a wearable medical device, or other external medical device. An AMD can sense cardiac electrical or mechanical activities via sensing electrodes and/or physiologic sensors, and detect cardiac events such as cardiac arrhythmia, or heart failure events indicative of worsening of heart failure (WHF). The AMD may include a pulse generator capable of generating and delivering electrostimulation therapy to the heart or other excitable tissue (e.g., neural targets) to restore or improve cardiac performance or to correct cardiac arrhythmia.

An AMD can receive acceleration information from a heat of a patient, such as HS information or other acceleration information via an accelerometer sensor or an acoustic sensor. The sensor can be included inside a housing of the AMD, or mounted on a catheter or a lead and communicatively coupled to the AMD. Heart sounds are associated with mechanical vibration of a heart and blood flow through the heart. Heart sounds recur with each cardiac cycle and are separated and classified according to the activity associated with the vibration. Typically, heart sounds sensed from a subject may include several components within a cardiac cycle, including a first (S1), a second (S2), a third (S3), or a fourth (S4) heart sound. S1 is associated with the vibrational sound made by the heart during tensing of the mitral valve. S2 is produced by closure of the aortic and pulmonary valves, and marks the beginning of diastole. S3 is an early diastolic sound corresponding to passive ventricular filling during diastole, when the blood rushes into the ventricles. S4 is a late diastolic sound corresponding to active ventricular filling when the atria contract and push the blood into the ventricles. In a healthy subject, S3 is usually faint and S4 is rarely audible. A pathologic S3 or S4 may be higher pitched and louder.

Heart sounds can be used to detect various cardiac events. For example, S1 and/or S2 may be used to detect cardiac arrhythmia such as supraventricular tachycardia or ventricular tachycardia. Pulmonary fluid accumulation in CHF patients may cause elevated ventricular filling pressure and diastolic dysfunction, resulting in pathologically louder S3. Forceful atrial contraction to overcome an abnormally stiff ventricle in a CHF patient may produce profound S4. Therefore, monitoring S3 or S4 may help determining patient diastolic dysfunction, detecting a WHF event, or assessing patient risk of developing future WHF.

Disclosed herein are systems, devices, and methods for monitoring and assessing the function of an implanted PHV using heart sounds generated by the PHV. Assessment of PHV function can be carried out during a heart valve replacement procedure, or during patient post-operative recovery and thereafter. The subject matter discussed in this document can improve the technology of prosthetic valve replacement. For example, peri-operative monitoring of PHV function can help identify an optimal position to anchor the PHV, such as one that corresponds to an increased S2 intensity and indicates a desired pressure gradient from the aorta to the left ventricle. Compared to conventional post-operative follow-up visits and evaluation, ambulatory post-operative monitoring of PHV function, such as via an ambulatory medical device discussed herein, can detect early signs of PHV dysfunction or failure associated with malpositioning or valve migration/embolization. The HS information (e.g., S2 intensity) produced by the PHV can be easily obtained and accurately reflect the PHV functionality and track patient progress in recovery. With the early detection of PHV dysfunction, a physician can take early intervention to prevent or reduce post-operative adverse events or complications, such as conversion to open surgery, renal failure, need for pacemaker implantation, stroke, or myocardial infarction, among others.

According to some other embodiments, the present medical-device system can be configured to monitor patient heart failure status, detect early signs of worsening heart failure (WHF) attributable to a dysfunctional PHV, alert caregivers of such conditions, and provide device therapy to correct the deteriorated cardiac function or slow down the deterioration. As such, the present subject matter can improve patient outcome and reduce the overall healthcare cost.

Another aspect of the present subject matter relates to risk stratification of patients with regurgitation and/or stenosed heart valves. Ambulatory HS monitoring via the systems and methods discussed in this document can be used to screen patients with early signs of valvular heart disease (e.g., aortic stenosis). The patients may be further identified as candidates for heart valve replacement (e.g., SAVR or TAVR). Therefore, with the system and methods discussed herein, more patients may benefit from prosthetic heart valves and valve replacement procedures.

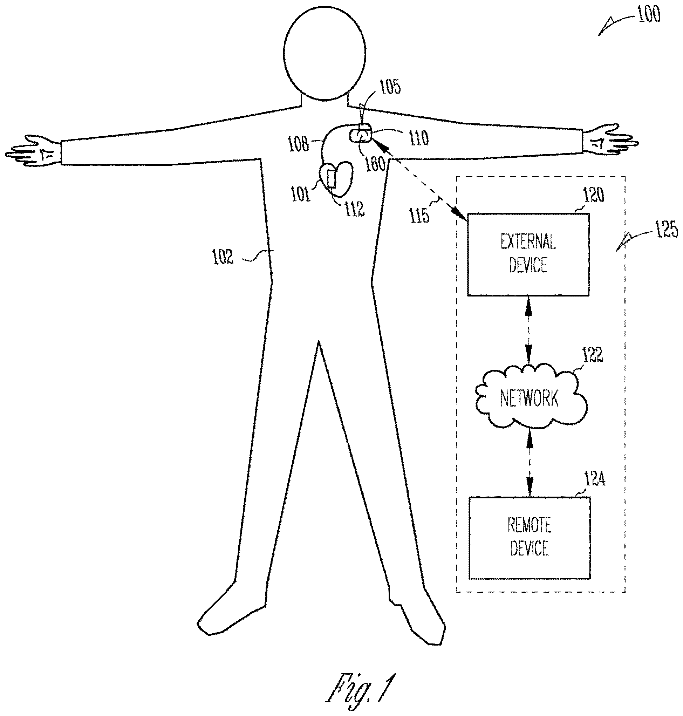

FIG. 1 illustrates generally an example of a patient management system 100 and portions of an environment in which the system 100 may operate. The patient management system 100 may perform a range of activities, including remote patient monitoring and diagnosis of a disease condition. Such activities may be performed proximal to a patient, such as in the patient's home or office, through a centralized server, such as in a hospital, clinic or physician's office, or through a remote workstation, such as a secure wireless mobile computing device.

The patient management system 100 may include an ambulatory system 105 associated with a patient 102, an external system 125, and a telemetry link 115 providing for communication between the ambulatory system 105 and the external system 125.

The ambulatory system 105 may include an ambulatory medical device (AMD) 110. In an example, the AMD 110 may be an implantable device subcutaneously implanted in a chest, abdomen, or other parts of the patient 102. Examples of the implantable device may include, but are not limited to, pacemakers, pacemaker/defibrillators, cardiac resynchronization therapy (CRT) devices, cardiac remodeling control therapy (RCT) devices, neuromodulators, drug delivery devices, biological therapy devices, diagnostic devices such as cardiac monitors or loop recorders, or patient monitors, among others. The AMD 110 alternatively or additionally may include a subcutaneous medical device such as a subcutaneous monitor or diagnostic device, external monitoring or therapeutic medical devices such as automatic external defibrillators (AEDs) or Holter monitors, or wearable medical devices such as patch-based devices, smart watches, or smart accessories.

By way of example, the AMD 110 may be coupled to a lead system 108. The lead system 108 may include one or more transvenously, subcutaneously, or non-invasively placed leads or catheters. Each lead or catheter may include one or more electrodes. The arrangements and uses of the lead system 108 and the associated electrodes may be determined using the patient need and the capability of the AMD 110. The associated electrodes on the lead system 108 may be positioned at the patient's thorax or abdomen to sense a physiologic signal indicative of cardiac activity, or physiologic responses to diagnostic or therapeutic stimulations to a target tissue. By way of example and not limitation, and as illustrated in FIG. 1, the lead system 108 may be surgically inserted into, or positioned on the surface of, a heart 101. The electrodes on the lead system 108 may be positioned on a portion of a heart 101, such as a right atrium (RA), a right ventricle (RV), a left atrium (LA), or a left ventricle (LV), or any tissue between or near the heart portions. In some examples, the lead system 108 and the associated electrodes may alternatively be positioned on other parts of the body to sense a physiologic signal containing information about patient heart rate or pulse rate. In an example, the ambulatory system 105 may include one or more leadless sensors not being tethered to the AMD 110 via the lead system 108. The leadless ambulatory sensors may be configured to sense a physiologic signal and wirelessly communicate with the AMD 110.

The AMD 110 may be configured as a monitoring and diagnostic device. The AMD 110 may include a hermetically sealed can that houses one or more of a data receiver circuit, a control circuit, a communication circuit, and a battery, among other components. The data receiver circuit may sense a physiologic signal, such as by using a physiologic sensor or the electrodes associated with the lead system 108. Examples of the physiologic signal may include one or more of electrocardiogram, intracardiac electrogram, arrhythmia, heart rate, heart rate variability, intrathoracic impedance, intracardiac impedance, arterial pressure, pulmonary artery pressure, left atrial pressure, right ventricular (RV) pressure, left ventricular (LV) coronary pressure, coronary blood temperature, blood oxygen saturation, one or more heart sounds, intracardiac acceleration, physical activity or exertion level, physiologic response to activity, posture, respiratory rate, tidal volume, respiratory sounds, body weight, or body temperature.

In an example, the AMD 110 may include a heart sounds (HS)-based event detector circuit 160 that can detect a physiologic event using patient HS information. Examples of the detected events may include cardiac arrhythmia episode, a worsening heart failure (WHF) event, or other cardiovascular or neurological events. The HS information may be sensed using a HS sensor (e.g., an accelerometer or acoustic sensor), and include vibrational or acoustic information produced by a prosthetic heart valve (PHV) 112. By way of example, the PHV 112 may include prosthetic aortic valve, prosthetic mitral valve, prosthetic tricuspid valve, and prosthetic pulmonary valve. The PHV 112 may be placed via an open-heart surgery or a transcatheter approach. The HS-based event detector circuit 160 can generate an indicator of function and viability of the PHV 112. The indicator may be presented to a clinician, who may take early intervention to prevent or reduce severe post-operative adverse events or complications. Additionally, the HS-based event detector circuit 160 may detect a cardiac event, such as WHF attributable to dysfunctional PHV. In an example, at least a portion of the functions of the HS-based event detector circuit 160, such as HS reconstruction or cardiac event detection, may be implemented in and executed by the external system 125.

The AMD 110 may additionally include a therapy unit that may generate and deliver one or more therapies. The therapy may be delivered to the patient 102 via the lead system 108 and the associated electrodes. The therapies may include electrical, magnetic, or other types of therapy. Examples of the anti-arrhythmic therapy may include pacing, cardioversion, defibrillation, neuromodulation, drug therapies, or biological therapies, among other types of therapies. In an example, the therapy unit may be configured to deliver cardiac resynchronization therapy (CRT) or multisite pacing for rectifying dyssynchrony and improving cardiac function in CHF patients. In another example, the therapy unit may be configured to deliver anti-arrhythmic therapy to treat arrhythmias. In yet another example, the therapy unit may be a drug delivery system, such as a drug infusion pump, configured to deliver one or more medications to the patient to treat CHF, arrhythmias, or other physiologic events.

The external system 125 may include a dedicated hardware/software system such as a programmer, a remote server-based patient management system, or alternatively a system defined predominantly by software running on a standard personal computer. The external system 125 may manage the patient 102 through the AMD 110 connected to the external system 125 via a communication link 115. This may include, for example, programming the AMD 110 to perform one or more of acquiring physiologic data (e.g., HS data), performing at least one self-diagnostic test (such as for a device operational status), generating an indicator of PHV function, detecting a target physiologic event (e.g., arrhythmia or WHF), or delivering or adjusting a therapy to the patient 102. Additionally, the external system 125 may receive device data from the AMD 110 via the communication link 115. Examples of the device data received by the external system 125 may include real-time or stored physiologic data from the patient 102, diagnostic data such as detection of WHF events, responses to therapies delivered to the patient 102, or device operational status of the AMD 110 (e.g., battery status and lead impedance). The telemetry link 115 may be an inductive telemetry link, a capacitive telemetry link, or a radiofrequency (RF) telemetry link, or wireless telemetry based on, for example, "strong" Bluetooth or IEEE 802.11 wireless fidelity "WiFi" interfacing standards. Other configurations and combinations of patient data source interfacing are possible.

By way of example and not limitation, the external system 125 may include an external device 120 in proximity of the AMD 110, and a remote device 124 in a location relatively distant from the AMD 110 in communication with the external device 120 via a telecommunication network 122. Examples of the external device 120 may include a programmer device.

The remote device 124 may be configured to evaluate collected patient data and provide alert notifications, among other possible functions. In an example, the remote device 124 may include a centralized server acting as a central hub for collected patient data storage and analysis. The server may be configured as a uni-, multi- or distributed computing and processing system. The remote device 124 may receive patient data from multiple patients including, for example, the patient 102. The patient data may be collected by the AMD 110, among other data acquisition sensors or devices associated with the patient 102. The server may include a memory device to store the patient data in a patient database. The server may include an alert analyzer circuit to evaluate the collected patient data to determine if specific alert condition is satisfied. Satisfaction of the alert condition may trigger a generation of alert notifications, such an alert of dysfunction of failure of the implanted PHV 112, deterioration of patient cardiac hemodynamic function, peri-operative position adjustment of the PHV 112, or post-operative WHF events, etc. In some examples, the alert conditions alternatively or additionally may be evaluated by the AMD 110. By way of example, alert notifications may include a Web page update, phone or pager call, E-mail, SMS, text or "Instant" message, as well as a message to the patient and a simultaneous direct notification to emergency services and to the clinician. Other alert notifications are possible.

The remote device 124 may additionally include one or more locally configured clients or remote clients securely connected over the network 122 to the server. Examples of the clients may include personal desktops, notebook computers, mobile devices, or other computing devices. System users, such as clinicians or other qualified medical specialists, may use the clients to securely access stored patient data assembled in the database in the server, and to select and prioritize patients and alerts for health care provisioning.

The network 122 may provide wired or wireless interconnectivity. In an example, the network 122 may be based on the Transmission Control Protocol/Internet Protocol (TCP/IP) network communication specification, although other types or combinations of networking implementations are possible. Similarly, other network topologies and arrangements are possible.

The external system 125, such as the external device 120 or the remote device 124, may output the detected physiologic events, such as an event of WHF, to a system user such as the patient or a clinician, or to a process including, for example, an instance of a computer program executable in a microprocessor. The process may include an automated generation or adjustment of therapy and patient management recommendations. The external system 125 may include respective display units for displaying the physiologic signals, or alerts, alarms, emergency calls, or other forms of warnings to signal the detection of a target physiologic event. The external system 125 may additionally display signal analysis results, such as the reconstructed HS segment, the detected physiologic event, or therapy and patient management recommendations, among other information.

Portions of the AMD 110 or the external system 125 may be implemented using hardware, software, firmware, or combinations thereof. Portions of the AMD 110 or the external system 125 may be implemented using an application-specific circuit that may be constructed or configured to perform one or more particular functions, or may be implemented using a general-purpose circuit that may be programmed or otherwise configured to perform one or more particular functions. Such a general-purpose circuit may include a microprocessor or a portion thereof, a microcontroller or a portion thereof, or a programmable logic circuit, a memory circuit, a network interface, and various components for interconnecting these components. For example, a "comparator" may include, among other things, an electronic circuit comparator that may be constructed to perform the specific function of a comparison between two signals or the comparator may be implemented as a portion of a general-purpose circuit that may be driven by a code instructing a portion of the general-purpose circuit to perform a comparison between the two signals.

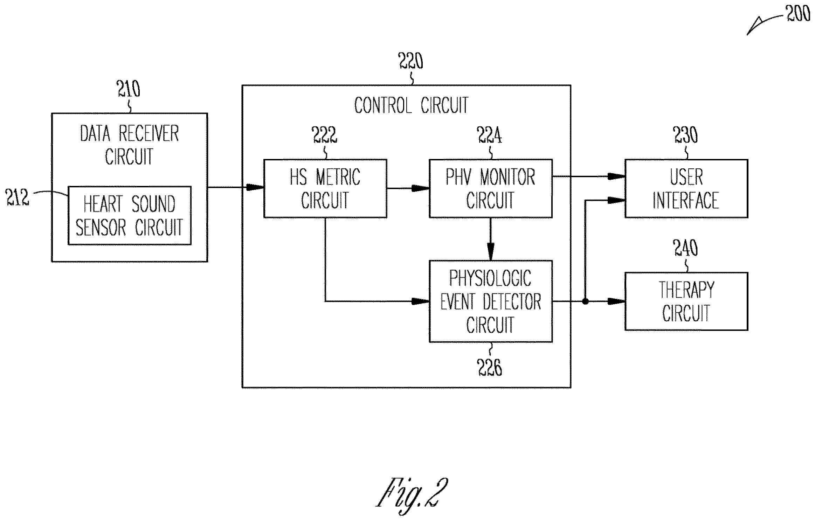

FIG. 2 illustrates generally an example of a PHV monitoring system 200 configured to monitor and assess function of an implanted PHV. The system 200 may be used during a heart valve replacement procedure (e.g., a SAVR or TAVR procedure) to generate peri-operative feedback to the surgeon that can assist in position adjustment of the PHV. The system 200 may also be used to monitor patient post-operative recovery progress and chronic cardiac function. In some examples, the system 200 may detect physiologic events, such as cardiac arrhythmia or worsening heart failure (WHF), based at least on the HS information.

The physiologic event detection system 200 may include one or more of a data receiver circuit 210, a control circuit 220, a user interface 230, and a therapy circuit 240. In an example, at least a portion of the physiologic event detection system 200 may be implemented in the AMD 110, the external system 125 such as one or more of the external device 120 or the remote device 124, or distributed between the AMD 110 and the external system 125.

The data receiver circuit 210 may receive physiologic information from a patient. The data receiver circuit 210 may include a sense amplifier circuit configured to sense a physiologic signal from a patient via a physiologic sensor, such as an implantable, wearable, or otherwise ambulatory sensor or electrodes associated with the patient. The sensor may be incorporated into, or otherwise associated with an ambulatory device such as the AMD 110. In some examples, the physiologic signals sensed from a patient may be stored in a storage device, such as an electronic medical record (EMR) system. The data receiver circuit 210 may receive the physiologic signal from the storage device, such as in response to a user command or a triggering event. Examples of the physiologic signals may include surface electrocardiography (ECG) sensed from electrodes placed on the body surface, subcutaneous ECG sensed from electrodes placed under the skin, intracardiac electrogram (EGM) sensed from the one or more electrodes on the lead system 108, heart rate signal, physical activity signal, or posture signal, a thoracic or cardiac impedance signal, arterial pressure signal, pulmonary artery pressure signal, left atrial pressure signal, RV pressure signal, LV coronary pressure signal, coronary blood temperature signal, blood oxygen saturation signal, heart sound signal, physiologic response to activity, apnea hypopnea index, one or more respiration signals such as a respiratory rate signal or a tidal volume signal, brain natriuretic peptide (BNP), blood panel, sodium and potassium levels, glucose level and other biomarkers and bio-chemical markers, among others. The data receiver circuit 210 may include one or more sub-circuits to digitize, filter, or perform other signal conditioning operations on the received physiologic signal.

In an example, the data receiver circuit 210 may include a heart sound (HS) sensor circuit 212 configured to receive HS information, such as a HS signal sensed from the patient. The HS information can include vibrational or acoustic information generated by the PHV 112, such as heart vibration caused by the closure of a prosthetic aortic valve at the end of systole and the beginning of diastole. In an example, the HS sensor circuit 212 may be coupled to a heart sound sensor to sense a body motion/vibration signal indicative of cardiac vibration, which is correlated to or indicative of heart sounds. The HS sensor may take the form of an accelerometer, an acoustic sensor, a microphone, a piezo-based sensor, or other vibrational or acoustic sensors. The accelerometer can be a one-axis, a two-axis, or a three-axis accelerometer. Examples of the accelerometer may include flexible piezoelectric crystal (e.g., quartz) accelerometer or capacitive accelerometer, fabricated using micro electromechanical systems (MEMS) technology. The HS sensor may be included in the AMD 110, disposed on a lead such as a part of the lead system 108, or mounted on a catheter used for delivering the PHV 112 to a target implant site. In some examples, the HS sensor may be included in a separate device than the AMD 110, such as a personal wearable device (e.g., smart phone or smart wearables) or an electronic stethoscope. In an example, the accelerometer may sense an epicardial or endocardial acceleration (EA) signal from a portion of a heart, such as on an endocardial or epicardial surface of one of a left ventricle, a right ventricle, a left atrium, or a right atrium. The EA signal may contain components corresponding to various HS components.

The control circuit 220 may reconstruct heart sounds from multiple epochs of low-rate HS signal data. The control circuit 220 be implemented as a part of a microprocessor circuit, which may be a dedicated processor such as a digital signal processor, application specific integrated circuit (ASIC), microprocessor, or other type of processor for processing information including physical activity information. Alternatively, the microprocessor circuit may be a processor that may receive and execute a set of instructions of performing the functions, methods, or techniques described herein.

The control circuit 220 may include circuit sets comprising one or more other circuits or sub-circuits, such as a HS metric circuit 222, a PHV monitor circuit 224, and a physiologic event detector circuit 226. These circuits may, alone or in combination, perform the functions, methods, or techniques described herein. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

The HS metric circuit 222 may include a signal conditioning circuit that, among other things, amplifying and filtering the received HS signal. In an example, the HS signal may be band-pass filtered to a frequency range of approximately 5-90 Hz, or approximately 9-90 Hz. In an example, the filter may include a double or higher-order differentiator configured to calculate a double or higher-order differentiation of the heart sound signal. The HS metric circuit 222 may compute an ensemble average of the filtered HS signal over multiple cardiac cycles, or over a specified time period, and detect one or more HS components, including a first (S 1) heart sound, a second (S2) heart sound, a third (S3) heart sound, or a fourth (S4) heart sound using respective time windows. The HS metric circuit 222 may generate a HS metric using the detected HS components. Examples of the HS metric may include an intensity (e.g., amplitude or signal energy under the curve) of a HS component, morphology or duration of one or more HS components, or one or more cardiac timing intervals. Examples of the cardiac timing intervals can include timing of S 1 or S2 relative to a fiducial point, a pre-ejection period (PEP) such as measured between the onset of the QRS to the S1 heart sound, a systolic timing interval (STI) such as measured between the onset of the QRS complex on the ECG to the S2 heart sound, a left-ventricular ejection time (LVET) such as measured as an interval between S 1 and S2 heart sounds, or a diastolic timing interval (DTI) such as measured between the S2 heart sound and the onset of the subsequent QRS complex on the ECG, among others. These HS-based cardiac timing intervals may be correlated with cardiac contractility or cardiac diastolic function of the heart. The HS metrics may further include PEP/LVET ratio, STI/DTI ratio, STI/cycle length (CL) ratio, or DTI/CL ratio, or other composite metrics.

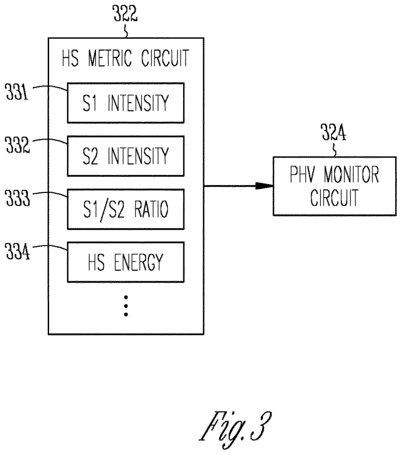

The PHV monitor circuit 224 may generate an indicator of PHV function using one or more HS metrics generated by the HS metric circuit 222. Dysfunction of an implanted PHV, such as a bioprosthetic aortic valve, may include, among other things, obstruction or insufficient opening during systole (which resembles stenosed native aortic valve), or insufficient closing during diastole causing blood leaking back to the left ventricle (which resembles aortic regurgitation such as due to a floppy aortic valve). The PHV indicator can be generated and presented in real time to a surgeon during a valve replacement procedure. The surgeon can use the PHV indicator as a feedback to control the positioning of the PHV during the procedure. In an example, an optimal position for the prosthetic valve can be determined along a direction corresponding to specified changes in a HS metric, such as an increase in S2 intensity. Additionally or alternatively, during patient post-operative recovery, the PHV indicator can be evaluated regularly (e.g., according to a specified frequency) to determine a rate of recovery and benefits gained from heart valve replacement. In an example, the PHV monitor circuit 224 may generate a trend of HS metric over time after the valve replacement. A change in the HS metric, or a rate of change in the HS metric, can be determined and indicate whether the progress in recovery is satisfactory or otherwise delayed. For example, if an amount of increase or a rate of increase of S2 intensity falls below a predetermined threshold, an alert of delayed post-implant recovery can be presented to a clinician, such as via the user interface 230. In some examples, the system 200 may also detect post-operative adverse events, such as cardiac arrhythmia or a worsening heart failure (WHF). Examples of HS metrics, and evaluation of PHV function using the HS metrics, are discussed below with reference to FIG. 3.

The physiologic event detector circuit 226 may be configured to detect a target physiologic event using HS information, among other physiologic information acquired from the patient. In an example, the physiologic event detector circuit 226 may generate a cardiac function indicator indicating myocardial contractility, cardiac synchrony, and cardiac hemodynamics, using one or more HS metrics. In an example, the physiologic event detector circuit 226 may detect a cardiac arrhythmia episode, or distinguish between different arrhythmias (e.g., atrial tachyarrhythmia, supraventricular tachyarrhythmia, or ventricular tachyarrhythmia) using one or more of S1 intensity, S2 intensity, or a measure of STI. For example, a reduction in S1 intensity may be indicative of reduced cardiac contractility, and a reduction in S2 intensity may be indicative of reduced cardiac output, both of which may be used to detect cardiac arrhythmias and deterioration of cardiac hemodynamics during arrhythmia. In another example, the HS metrics, such as S3 intensity, may be used to detect WHF. An increase in S3 intensity indicates reduced ventricular compliance and deteriorating diastolic function, signifying occurrence of WHF. Additionally or alternatively, a reduction in S1 intensity, or a reduction in STI may indicate poor cardiac contractility or reduced electromechanical coupling, which signifies occurrence of WHF. In an example, the physiologic event detector circuit 226 may detect a WHF event attributable to dysfunctional PHV using the HS metric and the PHV indicator, such as an increase in S3 intensity accompanied by a decrease in S2 intensity from their respective baselines. The physiologic event detector circuit 226 may additionally or alternatively detect respiratory, renal, neurological, among other medical conditions, based on the HS metrics.

The user interface 230 may include an input unit and an output unit. In an example, at least a portion of the user interface 230 may be implemented in the external system 125. The input unit may receive user input for programming the data receiver circuit 210 and the control circuit 220. The input unit may include a keyboard, on-screen keyboard, mouse, trackball, touchpad, touch-screen, or other pointing or navigating devices. The output unit may include a display for displaying the sensed HS signal, the generated HS metrics, the PHV indicator, and information about the detected physiologic events such as a WHF event, and any intermediate measurements or computations, among others. The output unit may also present to a user, such as via a display unit, the therapy titration protocol and recommended therapy, including a change of parameters in the therapy provided by an implanted device, the prescription to get a device implanted, the initiation or change in a drug therapy, or other treatment options of a patient. The output unit may include a printer for printing hard copies of information that may be displayed on a display unit. The signals and information may be presented in a table, a chart, a diagram, or any other types of textual, tabular, or graphical presentation formats. The presentation of the output information may include audio or other media format. In an example, the output unit may generate alerts, alarms, emergency calls, or other forms of warnings to signal the system user about the detected medical events.

The therapy circuit 240 may be configured to deliver a therapy to the patient, such as in response to the detected physiologic event. The therapy may be preventive or therapeutic in nature such as to modify, restore, or improve patient neural, cardiac, or respiratory functions. Examples of the therapy may include electrostimulation therapy delivered to the heart, a nerve tissue, other target tissues, a cardioversion therapy, a defibrillation therapy, or drug therapy including delivering drug to the patient. In some examples, the therapy circuit 240 may modify an existing therapy, such as adjust a stimulation parameter or drug dosage.

FIG. 3 illustrates by way of example and not limitation, an embodiment of portions of the system 200 that can generate a PHV indicator using various HS metrics. The HS metric circuit 322, which is an embodiment of the HS metric circuit 222, can generate from the HS signal one or more of an S1 intensity 331, S2 intensity 332, S1/S2 ratio 333, HS energy 334 at specific frequency band, among other HS metrics. S1 intensity 331, such as S1 amplitude or S1 signal energy (measured in time or frequency domain), is correlated with myocardial contractility. The S1 intensity 331 may include a first component corresponding to mitral valve closure and a second component corresponding to tricuspid valve closure. An increase in S1 intensity indicates an increase in contractility, and a decrease in left ventricular end-diastolic pressure (LVEDP). A dysfunctional PHV may cause regurgitation, thereby an elevated LVEDP. The PHV monitor circuit 324 may generate an indicator indicating a dysfunctional PHV if the S1 intensity falls below an S1 threshold.

S2 intensity 332, such as S2 amplitude or S2 signal energy (measured in time or frequency domain), is correlated with a pressure gradient across the aortic valve at the time of aortic valve closure. The S2 intensity 332 may include a first component corresponding aortic valve closure and a second component corresponding to pulmonary valve closure. A properly functional prosthetic aortic valve can prevent regurgitation, and establish adequate pressure gradient across the valve, represented by an increase in S2 intensity. Conversely, a decrease in S2 intensity may be a sign of PHV dysfunction due to regurgitation, which may lead to an elevated LVEDP and reduced trans-aortic valve pressure gradient. The PHV monitor circuit 324 may generate an indicator indicating PHV dysfunction if the S2 intensity falls below an S2 threshold.

The PHV monitor circuit 324 may generate a PHV indicator using a composite HS metric, such as S1/S2 ratio 333 computed as a ratio of an S1 intensity to an S2 intensity. In an example, the PHV monitor circuit 324 can generate a PHV indication indicating PHV dysfunction if the S1/S2 ratio exceeds a threshold or is outside of a specific value range.

In some examples, the HS metric circuit 322 may filter the HS signal, or a portion of the HS signal (e.g., a HS data window within a cardiac cycle), within a frequency band, and generate a HS energy 334 using the filtered HS signal. In an example, the HS signal can be filtered using a high-pass filter with a lower cutoff frequency of substantially 100 Hz. Generally, a HS signal produced by a properly functional PHV has substantially lower energy above 100 Hz. A dysfunctional bioprosthetic aortic value may introduce regurgitation, resulting in changes in HS that resemble aortic stenosis or regurgitation. This can be characterized by a broader HS frequency bands and excess energy above 100 Hz. The PHV monitor circuit 324 may generate an indicator of PHV dysfunction if the signal energy of the filtered HS signal (e.g., high-frequency HS components above 100 Hz) exceeds a threshold.

Other HS metrics, such as morphology or duration of a HS component, can also be used to assess PHV function. In an example, an increase in S2 duration may be indicative of regurgitation as a result of PHV dysfunction. Physiologic signals other than HS may also be used by the PHV monitor 324 to generate the PHV indicator. In an example, the data receiver circuit 210 may receive thoracic or cardiac impedance of the patient, and the PHV monitor circuit 324 may produce the indicator of PHV function further using the received thoracic or cardiac impedance. For example, a PHV dysfunction is indicated if the thoracic or cardiac impedance falls below a threshold.

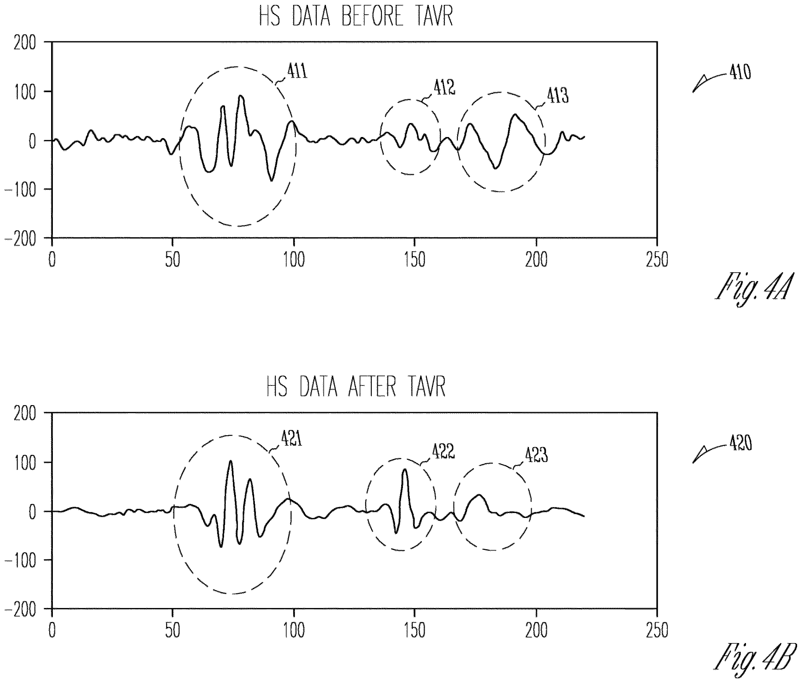

FIGS. 4A-4B are graphs illustrating changes in heart sounds of a patient subsequent to a TVAR procedure. The HS signals were recorded from the same patient using an accelerometer sensor. Specifically, FIG. 4A illustrates a portion of a HS signal 410, within a representative cardiac cycle, recorded before the TVAR procedure, and FIG. 4B illustrates a portion of a HS signal 420, within a representative cardiac cycle, recorded approximately two months after the TVAR procedure. The vertical axis represents amplitude of the HS signal (units of acceleration), and the horizontal axis represents samples of the signal over time. Various HS components, including S1411, S2 412, and S3 413 of the HS signal portion 410, and S1 421, S2 422, and S3 423 of the HS signal portion 420, can be detected such as by the HS metric circuit 222. Compared to pre-TAVR HS signal 410, the post-TAVR S2 422 has substantially increased from the pre-TAVR S2 412. Based at least on the increase in S2 422 from S2 412, the PHV monitor circuit 224 may generate a PHV indicator indicating a proper function of the prosthetic aortic valve. Additionally, post-TAVR S3 423 has substantially decreased from the pre-TAVR S3 413. The physiologic event detector circuit 226 may determine that the patient heart failure status has improved based at least on the decrease in S3 423 from the pre-TAVR S3 413, which can be associated with improved cardiac performance attributable to proper functioning of the prosthetic aortic valve.

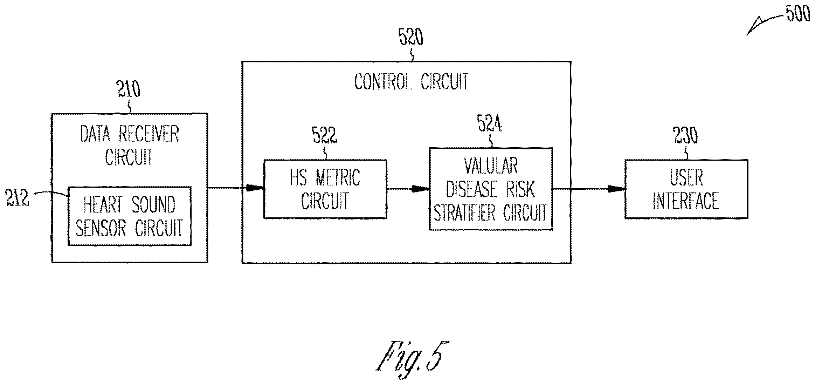

FIG. 5 illustrates generally an example of a patient monitoring system 500 configured to monitor and assess patient natural valve function. The system 500 may include an ambulatory medical device, such as the AMD 110, configured to identify patients at risk of a valvular disease (e.g., stenosis or regurgitation), and to determine patient candidacy for heart valve repair or replacement (e.g., a SAVR or TAVR procedure). Similar to the system 200, the system 500 can include a data receiver circuit 210 and a control circuit 520. The data receiver circuit 210 can be configured to receive physiologic data, such as heart sound (HS) information received by the heart sound sensor circuit 212. The received HS information includes vibrational or acoustic information generated by a natural heart valve, such as a natural stenosed aortic valve of the patient. The control circuit 520, which may be implemented as a microprocessor or a set of circuits or subcircuits, may include a HS metric circuit 522 and a valvular disease risk stratifier circuit 524. Similar to the HS metric circuit 220, the HS metric circuit 522 can generate a HS metric, such as an intensity (e.g., amplitude or signal energy under the curve), morphology, or duration of a HS component, or one or more cardiac timing intervals. However, instead of processing HS information produced by a PHV, the HS metric circuit 522 generates the HS metric using HS information produced by a natural heart valve.

The valvular disease risk stratifier circuit 524 can identify patients at risk of valvular heart disease (e.g., aortic stenosis), using the HS metrics generated by the HS metric circuit 522. In an example, the valvular disease risk stratifier circuit 524 can generate a risk indicator of elevated valvular disease risk if the S2 intensity does not satisfy a condition relative to a reference S2 intensity. In another example, a risk indicator of elevated valvular disease risk may be generated in response to an S1 intensity falling below a threshold. In yet another example, the valvular disease risk stratifier circuit 524 can stratify valvular disease risk using a composite HS metric. For example, an elevated valvular disease risk can be generated if a ratio of S1 intensity to S2 intensity exceeds a threshold. In another example, a risk indicator of elevated valvular disease risk may be generated if a HS signal energy in a specified frequency band (such as above 100 Hz) exceeds a threshold. The risk indicator can be output to a user interface 230. Patients having a risk indicator indicating an elevated risk of valvular disease may be further determined for candidacy for valve replacement procedures (e.g., SAVR or TAVR). The HS-based valvular disease risk stratification discussed herein can be implemented in an ambulatory medical device. Ambulatory monitoring of HS via the system 500 can help identify candidates for aortic valve replacement at an early stage before patient developing adverse events, such as WHF.

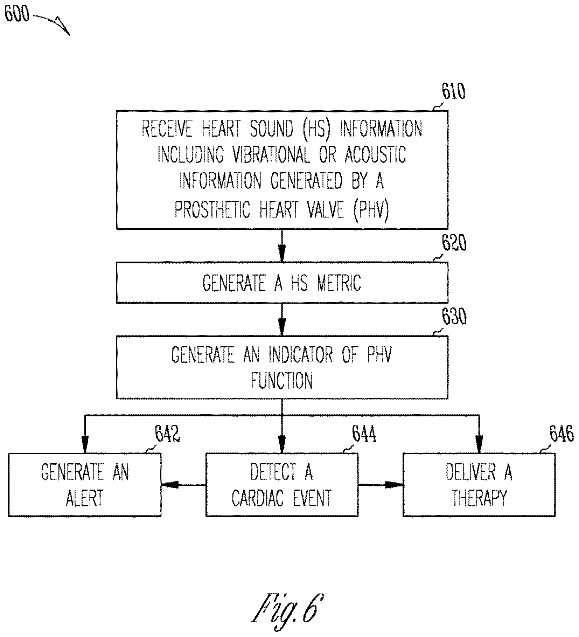

FIG. 6 is a flow chart illustrating an example of a method 600 for assessing a PHV implanted in a patient. The PHV can be a mechanical device or a bioprosthetic valve, such as a prosthetic aortic valve surgically implanted via an open-heart SAVR procedure, or via a minimally invasive TAVR procedure. The method 600 may be implemented and executed in an ambulatory medical device such as an implantable or wearable medical device, or in a remote patient management system. In an example, the method 600 may be implemented in and executed by the cardiac arrhythmia detection circuit 160 in the AMD 110, the external system 130, or the physiologic event detection system 200.

The method 600 commences at 610, where heart sound (HS) information may be received from a patient. The HS information may be sensed from a physiologic sensor circuit, or retrieved from a data storage device. Examples of the HS sensor may include an accelerometer, an acoustic sensor, a microphone, a piezo-based sensor, or other vibrational or acoustic sensors. The accelerometer can be a one-axis, a two-axis, or a three-axis accelerometer. The HS sensor may be included in the AMD 110, disposed on a lead such as a part of the lead system 108, or mounted on a catheter used for delivering the PHV 112 to a target implant site. The HS information can include vibrational or acoustic information generated by the PHV 112, such as vibration initiated by the closure of a prosthetic aortic valve at the end of systole and the beginning of diastole. In some examples, other physiologic information, other than HS, may be received at 610, such as cardiac electrical signal such as an electrocardiography (ECG) or an intracardiac electrogram (EGM), thoracic or cardiac impedance signal, arterial pressure signal, pulmonary artery pressure signal, left atrial pressure signal, RV pressure signal, LV pressure signal, physiologic response to activity, apnea hypopnea index, one or more respiration signals such as a respiration rate signal or a tidal volume signal, among others.

At 620, a HS metric may be generated, such as via the HS metric circuit 222. Examples of the HS metric may include an intensity (e.g., amplitude or signal energy under the curve) of a HS component, morphology or duration of one or more HS components, or one or more cardiac timing intervals, as discussed above with reference to FIG. 2.

At 630, a PHV indicator can be generated using one or more HS metrics, such as via the PHV monitor circuit 224. The PHV indicator indicates a function of the PHV. In an example, the PHV indicator may indicate a dysfunction of an implanted bioprosthetic aortic valve, such as obstruction or insufficient opening during systole, or insufficient closing during diastole causing blood leaking back to the left ventricle. One or more HS metrics may be used to generate such a PHV indicator. As illustrated in FIG. 3, a PHV indicator indicating PHV dysfunction may be generated in response to an S1 intensity falling below an S 1 threshold. In another example, PHV dysfunction may be indicated if an S2 intensity falls below an S2 threshold, as a decrease in S2 may be a sign of aortic regurgitation, which can result in an elevated LVEDP and reduced trans-aortic valve pressure gradient. In yet another example, the PHV indicator may be generated using a composite HS metric, such as a ratio of S 1 intensity to S2 intensity (S1/S2 ratio). PHV dysfunction is indicated if the S1/S2 ratio exceeds a threshold or is outside of a specific value range.

In some examples, the received HS information (e.g., a HS signal, or a portion of a HS signal such as a HS data window within a cardiac cycle), may be filtered through a filter circuit with a specified frequency passband, and signal energy of the filtered HS information may be used to generate the PHV indicator. In an example, the filter can be a high-pass filter with a lower cutoff frequency of substantially 100 Hz. For example, a dysfunctional bioprosthetic aortic value may result in changes in HS that resemble aortic stenosis or regurgitation, characterized by increased energy at frequencies above 100 Hz. A PHV dysfunction is indicated if the signal energy of the filtered HS signal exceeds a threshold.

The HS metric generated at 620 and the PHV indicator generated at 630 may be provided to a user or a process, such as one or more of 642, 644, or 646. At 642, an alert can be generated if the PHV function indicator indicating a PHV dysfunction. In an example, the HS metric and/or the PHV indicator can be presented as a real-time feedback to a surgeon during a valve replacement procedure (e.g., SAVR or TAVR). The surgeon can use the PHV indicator to optimize positioning of the PHV. The optimal position for anchoring the prosthetic valve can be along a direction corresponding to a specified change in a HS metric, such as an increase in S2 intensity. In another example, HS metric generation at 620 and PHV indicator generation at 630 can be carried out on a regular basis, such as according to a specified frequency during patient post-operative recovery phase, to assess a rate of recovery and benefits gained from the valve replacement surgery. At 642, an alert can be generated if a temporal change in the HS metric, or a rate of change of the HS metric, falls below a predetermined threshold, indicating a delayed post-implant recovery.

At 644, post-operative adverse events, such as cardiac arrhythmia or a worsening heart failure (WHF) event, may be detected using the HS metrics, such as via the physiologic event detector circuit 226. For example, an increase in S3 intensity indicates reduced ventricular compliance and deteriorating diastolic function, signifying occurrence of WHF. Additionally or alternatively, a reduction in S 1 intensity, or a reduction in STI may indicate poor cardiac contractility or reduced electromechanical coupling, which signifies occurrence of WHF. In an example, a WHF event attributable to dysfunctional PHV can be detected at 644 using the HS metric and the PHV indicator, such as an increase in S3 intensity accompanied by a decrease in S2 intensity from their respective baselines. Deterioration of patient cardiac hemodynamics, and any detected post-operative adverse events such as the WHF event, can be reported to a clinician, with an alert at 642. In some examples, a recommendation may be generated and provided to the user at 642. The recommendation may include one or more of further diagnostic tests to be performed, adjustment of one or more parameters for detecting the physiologic event, or therapy to be delivered or adjustment of one or more therapy parameters. The system user may review and adjudicate the detected physiologic event, and reprogram one or more detection or therapy parameters, such as using the user interface 230.

At 656, a therapy may be delivered to the patient in response to the detected physiologic event, such as via the therapy circuit 240 as illustrated in FIG. 2. Examples of the therapy may include electrostimulation therapy delivered to the heart, a nerve tissue, other target tissues, a cardioversion therapy, a defibrillation therapy, or drug therapy including delivering drug to a tissue or organ. In some examples, an existing therapy or treatment plan may be modified to treat the detected arrhythmia, such as modify patient follow-up schedule, or adjust a stimulation parameter or drug dosage.

In various examples, the method 600 may comprise steps of assess patient natural valve function and identify patient candidacy for PHV replacement procedures. The HS information received at 610 may include patient natural HS information generated by a natural heart valve. At 620, the HS metric may include a natural HS metric generated from the natural HS information. A risk indicator can be generated using the natural HS metric, such as via the valvular disease risk stratifier circuit 524 as discussed above with reference to FIG. 5. 230. Patients having a risk indicator indicating an elevated risk of valvular disease may be further identified to receive valve replacement procedures (e.g., SAVR or TAVR). Ambulatory HS monitoring and risk stratification as discussed herein can help identify candidates for aortic valve replacement at an early stage before patient developing adverse events, such as WHF.

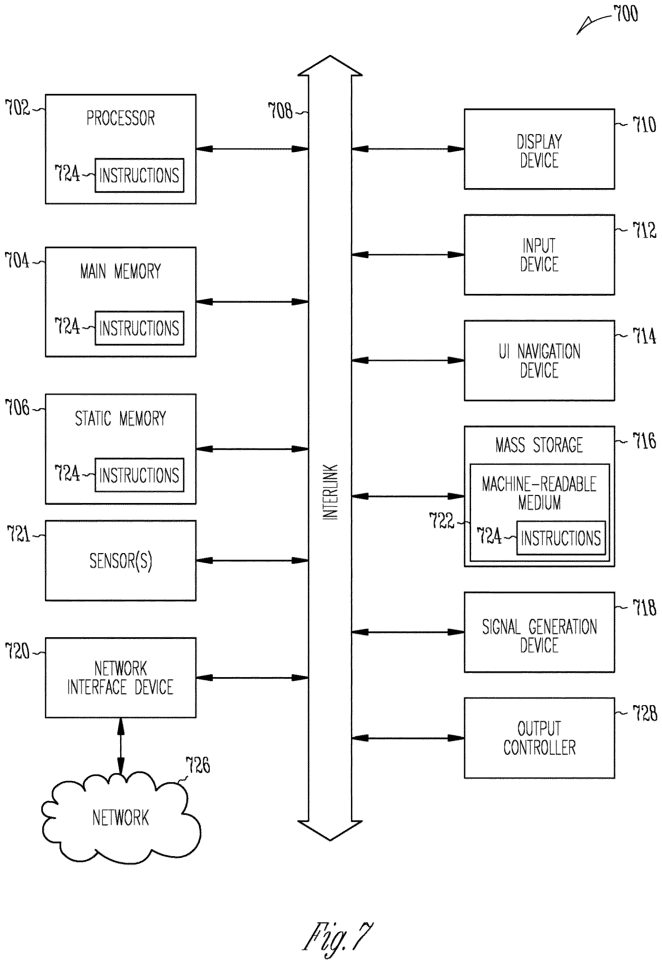

FIG. 7 illustrates generally a block diagram of an example machine 700 upon which any one or more of the techniques (e.g., methodologies) discussed herein may perform. Portions of this description may apply to the computing framework of various portions of the LCP device, the IMD, or the external programmer.

In alternative embodiments, the machine 700 may operate as a standalone device or may be connected (e.g., networked) to other machines. In a networked deployment, the machine 700 may operate in the capacity of a server machine, a client machine, or both in server-client network environments. In an example, the machine 700 may act as a peer machine in peer-to-peer (P2P) (or other distributed) network environment. The machine 700 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 700 may include a hardware processor 702 (e.g., a central processing unit (CPU), a graphics processing unit (GPU), a hardware processor core, or any combination thereof), a main memory 704 and a static memory 706, some or all of which may communicate with each other via an interlink (e.g., bus) 708. The machine 700 may further include a display unit 710 (e.g., a raster display, vector display, holographic display, etc.), an alphanumeric input device 712 (e.g., a keyboard), and a user interface (UI) navigation device 714 (e.g., a mouse). In an example, the display unit 710, input device 712 and UI navigation device 714 may be a touch screen display. The machine 700 may additionally include a storage device (e.g., drive unit) 716, a signal generation device 718 (e.g., a speaker), a network interface device 720, and one or more sensors 721, such as a global positioning system (GPS) sensor, compass, accelerometer, or other sensor. The machine 700 may include an output controller 728, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 716 may include a machine readable medium 722 on which is stored one or more sets of data structures or instructions 724 (e.g., software) embodying or utilized by any one or more of the techniques or functions described herein. The instructions 724 may also reside, completely or at least partially, within the main memory 704, within static memory 706, or within the hardware processor 702 during execution thereof by the machine 700. In an example, one or any combination of the hardware processor 702, the main memory 704, the static memory 706, or the storage device 716 may constitute machine readable media.

While the machine-readable medium 722 is illustrated as a single medium, the term "machine readable medium" may include a single medium or multiple media (e.g., a centralized or distributed database, and/or associated caches and servers) configured to store the one or more instructions 724.

The term "machine readable medium" may include any medium that is capable of storing, encoding, or carrying instructions for execution by the machine 700 and that cause the machine 700 to perform any one or more of the techniques of the present disclosure, or that is capable of storing, encoding or carrying data structures used by or associated with such instructions. Nonlimiting machine-readable medium examples may include solid-state memories, and optical and magnetic media. In an example, a massed machine-readable medium comprises a machine readable medium with a plurality of particles having invariant (e.g., rest) mass. Accordingly, massed machine-readable media are not transitory propagating signals. Specific examples of massed machine-readable media may include: non-volatile memory, such as semiconductor memory devices (e.g., Electrically Programmable Read-Only Memory (EPROM), Electrically Erasable Programmable Read-Only Memory (EPSOM)) and flash memory devices; magnetic disks, such as internal hard disks and removable disks; magneto-optical disks; and CD-ROM and DVD-ROM disks.

The instructions 724 may further be transmitted or received over a communication network 726 using a transmission medium via the network interface device 720 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as WiFi^{®}, IEEE 802.16 family of standards known as WiMax^{®}), IEEE 802.15.4 family of standards, peer-to-peer (P2P) networks, among others. In an example, the network interface device 720 may include one or more physical jacks (e.g., Ethernet, coaxial, or phone jacks) or one or more antennas to connect to the communication network 726. In an example, the network interface device 720 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 700, and includes digital or analog communications signals or other intangible medium to facilitate communication of such software.

Various embodiments are illustrated in the figures above. One or more features from one or more of these embodiments may be combined to form other embodiments.

The method examples described herein can be machine or computer-implemented at least in part. Some examples may include a computer-readable medium or machine-readable medium encoded with instructions operable to configure an electronic device or system to perform methods as described in the above examples. An implementation of such methods may include code, such as microcode, assembly language code, a higher-level language code, or the like. Such code may include computer readable instructions for performing various methods. The code can form portions of computer program products. Further, the code can be tangibly stored on one or more volatile or non-volatile computer-readable media during execution or at other times.

The above detailed description is intended to be illustrative, and not restrictive. The scope of the disclosure should therefore be determined with references to the appended claims.

## Claims

1. A medical-device system (100), comprising:
a sensor circuit (212) configured to receive acceleration information of a heart of a patient, the acceleration information including a heart sound signal indicative of functioning of a prosthetic heart valve (PHV) in the patient; and
a PHV monitor circuit (224) configured to filter the heart sound signal using a filter with a lower cutoff frequency of substantially 100 Hz, to generate a heart sound (HS) metric using the filtered heart sound signal, and to generate an indicator of function of the PHV using the generated HS metric.

2. The system (100) of claim 1, wherein the PHV includes a bioprosthetic aortic valve implanted surgically in the patient or via a transcatheter approach.

3. The (100) system of any of claims 1-2, wherein the HS metric includes a second heart sound (S2) intensity.

4. The (100) system of any of claims 1-3, wherein the HS metric includes a first heart sound (S1) intensity.

5. The (100) system of any of claims 1-4, wherein the HS metric includes a HS ratio of a first heart sound (S1) intensity to a second heart sound (S2) intensity.

6. The system (100) of any of claims 1-5, wherein the PHV monitor circuit (224) is configured to generate the indicator indicating PHV dysfunction in responds to the generated HS metric exceeding a threshold or being outside of a specific range.

7. The system (100) of any of claims 1-6, wherein the PHV monitor circuit (224) is configured to:
generate the HS metric including signal energy of the filtered HS signal; and
generate the indicator indicating PHV dysfunction in responds to the generated signal energy of the filtered HS signal exceeding a threshold.

8. The system (100) of any of claims 1-7, comprising an output circuit configured to generate an alert if the PHV function indicator indicates a PHV dysfunction.

9. The system (100) of any of claims 1-8, wherein the output circuit is configured to generate an indication of position adjustment of the PHV during a heart valve replacement procedure using the generated HS metric.

10. The system (100) of any of claims 1-9, wherein the PHV monitor circuit (224) is configured to generate a trend of the HS metric over time post implant of the PHV, and the system comprises an output circuit-configured to generate an alert of delayed post-implant recovery using the generated HS metric trend.

11. The system (100) of any of claims 1-10, comprising a heart failure output circuit configured to detect worsening heart failure (WHF) in the patient using the received acceleration information.

12. The system (100) of claim 11, wherein the heart failure detector circuit is configured to detect WHF attributable to PHV dysfunction in response to an increase in S3 intensity and a decrease in S2 intensity from their respective baselines.

13. The system (100) of any of claims 1-12, wherein the sensor circuit (212) is coupled to an accelerometer sensor mounted on a catheter used for delivering the PHV to a target implant site and configured to sense the acceleration information from the patient.

## Patentansprüche

1. Medizinisches Vorrichtungssystem (100), umfassend:
eine Sensorschaltung (212), die eingerichtet ist, Beschleunigungsinformationen bezüglich eines Herzens eines Patienten zu empfangen, wobei die Beschleunigungsinformationen ein Herztonsignal einschließen, das ein Funktionieren einer Herzklappenprothese (PHV) in dem Patienten anzeigt; und
eine PHV-Überwachungsschaltung (224), die eingerichtet ist, das Herztonsignal unter Verwendung eines Filters mit einer unteren Grenzfrequenz von im Wesentlichen 100 Hz zu filtern, unter Verwendung des gefilterten Herztonsignals eine Herzton(HS)-Metrik zu erzeugen und unter Verwendung der erzeugten HS-Metrik einen Indikator für eine Funktion der PHV zu erzeugen.

2. System (100) nach Anspruch 1, wobei die PHV eine Aortenklappenbioprothese einschließt, die dem Patienten chirurgisch oder mittels einer Transkathetermethode implantiert ist.

3. System (100) nach einem der Ansprüche 1-2, wobei die HS-Metrik eine Intensität eines zweiten Herztons (S2) einschließt.

4. System (100) nach einem der Ansprüche 1-3, wobei die HS-Metrik eine Intensität eines ersten Herztons (S1) einschließt.

5. System (100) nach einem der Ansprüche 1-4, wobei die HS-Metrik ein HS-Verhältnis einer ersten Intensität eines ersten Herztons (S1) zu einer Intensität eines zweiten Herztons (S2) einschließt.

6. System (100) nach einem der Ansprüche 1-5, wobei die PHV-Überwachungsschaltung (224) eingerichtet ist, als Reaktion darauf, dass die erzeugte HS-Metrik einen Schwellenwert überschreitet oder außerhalb eines spezifischen Bereichs liegt, den Indikator zu erzeugen, der eine PHV-Fehlfunktion anzeigt.

7. System (100) nach einem der Ansprüche 1-6, wobei die PHV-Überwachungsschaltung (224) eingerichtet ist zum:
Erzeugen der HS-Metrik, die eine Signalenergie des gefilterten HS-Signals einschließt; und
Erzeugen des Indikators, der eine PHV-Fehlfunktion anzeigt, als Reaktion darauf, dass die erzeugte Signalenergie des gefilterten HS-Signals einen Schwellenwert überschreitet.

8. System (100) nach einem der Ansprüche 1-7, eine Ausgabeschaltung umfassend, die konfiguriert ist, eine Warnung zu erzeugen, wenn der PHV-Funktionsindikator eine PHV-Fehlfunktion angibt.

9. System (100) nach einem der Ansprüche 1-8, wobei die Ausgabeschaltung konfiguriert ist, während eines Eingriffs, bei dem eine Herzklappe ausgetauscht wird, unter Verwendung der erzeugten HS-Metrik eine Angabe zu einer Positionsanpassung der PHV zu erzeugen.

10. System (100) nach einem der Ansprüche 1-9, wobei die PHV-Überwachungsschaltung (224) konfiguriert ist, nach der Implantation der PHV einen Trend der HS-Metrik im Zeitverlauf zu erzeugen, und das System eine Ausgabeschaltung umfasst, die konfiguriert ist, wegen einer verzögerten Erholung nach einer Implantation eine Warnung zu erzeugen.

11. System (100) nach einem der Ansprüche 1-10, eine Herzinsuffizienz-Ausgabeschaltung umfassend, die konfiguriert ist, unter Verwendung der empfangenen Beschleunigungsinformationen eine sich verschlechternde Herzinsuffizienz (WHF) bei dem Patienten zu detektieren.

12. System (100) nach Anspruch 11, wobei die Herzinsuffizienz-Detektorschaltung konfiguriert ist, als Reaktion auf einen Anstieg der S3-Intensität und eine Abnahme der S2-Intensität gegenüber ihren jeweiligen Grundlinien eine WHF zu detektieren, die einer PHV-Fehlfunktion zuzuschreiben ist.

13. System (100) nach einem der Ansprüche 1-12, wobei die Sensorschaltung (212) mit einem Beschleunigungssensor gekoppelt ist, der an einem Katheter montiert ist, der verwendet wird, um die PHV an einer Ziel-Implantationsstelle abzugeben, und der konfiguriert ist, die Beschleunigungsinformationen von dem Patienten zu erfassen.

## Revendications

1. Système de dispositif médical (100), comprenant :
un circuit de capteur (212) configuré pour recevoir une information d'accélération du coeur d'un patient, l'information d'accélération incluant un signal de bruits du coeur indicatif du fonctionnement d'une valvule cardiaque prothétique (PHV) implantée sur le patient ; et
un circuit de moniteur de PHV (224) configuré pour filtrer le signal de bruits du coeur en utilisant un filtre présentant une fréquence de coupure inférieure de sensiblement 100 Hz, afin de générer une mesure de bruits du coeur (HS) en utilisant le signal de bruits du coeur filtré, et afin de générer un indicateur de fonction de la PHV en utilisant la mesure de HS générée.

2. Système (100) selon la revendication 1, dans lequel la PHV inclut une valvule aortique bio-prothétique qui est implantée chirurgicalement chez le patient ou via une approche par transcathéter.

3. Système (100) selon l'une quelconque des revendications 1 et 2, dans lequel la mesure de HS inclut une seconde intensité de bruits du coeur (S2).

4. Système (100) selon l'une quelconque des revendications 1 à 3, dans lequel la mesure de HS inclut une première intensité de bruits du coeur (S1).

5. Système (100) selon l'une quelconque des revendications 1 à 4, dans lequel la mesure de HS inclut un rapport de HS égal à une première intensité de bruits du coeur (S1) sur une seconde intensité de bruits du coeur (S2).

6. Système (100) selon l'une quelconque des revendications 1 à 5, dans lequel le circuit de moniteur de PHV (224) est configuré pour générer l'indicateur qui indique un dysfonctionnement de PHV en réponse au fait que la mesure de HS générée excède un seuil ou est à l'extérieur d'une plage spécifique.

7. Système (100) selon l'une quelconque des revendications 1 à 6, dans lequel le circuit de moniteur de PHV (224) est configuré pour :
générer la mesure de HS qui inclut une énergie de signal du signal de HS filtré ; et
générer l'indicateur qui indique un dysfonctionnement de PHV en réponse au fait que l'énergie de signal générée du signal de HS filtré excède un seuil.

8. Système (100) selon l'une quelconque des revendications 1 à 7, comprenant un circuit de sortie configuré pour générer une alerte si l'indicateur de fonction de PHV indique un dysfonctionnement de PHV.

9. Système (100) selon l'une quelconque des revendications 1 à 8, dans lequel le circuit de sortie est configuré pour générer une indication de réglage de position de la PHV pendant une procédure de remplacement de valvule cardiaque en utilisant la mesure de HS générée.

10. Système (100) selon l'une quelconque des revendications 1 à 9, dans lequel le circuit de moniteur de PHV (224) est configuré pour générer une tendance de la mesure de HS en fonction du temps après l'implantation de la PHV, et le système comprend un circuit de sortie configuré pour générer une alerte de récupération post-implantaire retardée en utilisant la tendance de mesure de HS générée.

11. Système (100) selon l'une quelconque des revendications 1 à 10, comprenant un circuit de sortie d'insuffisance cardiaque configuré pour détecter une aggravation de l'insuffisance cardiaque (WHF) chez le patient en utilisant l'information d'accélération reçue.

12. Système (100) selon la revendication 11, dans lequel le circuit de détecteur d'insuffisance cardiaque est configuré pour détecter une WHF attribuable à un dysfonctionnement de PHV en réponse à une augmentation d'une intensité S3 et à une diminution d'une intensité S2 par rapport à leurs lignes de base respectives.

13. Système (100) selon l'une quelconque des revendications 1 à 12, dans lequel le circuit de capteur (212) est couplé à un capteur d'accéléromètre qui est monté sur un cathéter utilisé pour procéder à la pose de la PHV au niveau d'un site d'implantation cible et qui est configuré pour détecter l'information d'accélération à partir du patient.
